# EUROPEAN PATENT APPLICATION

(11) **EP 3 574 913 A1**
(43) Date of publication of application: **04.12.2019**
(21) Application number: 18744172.0
(22) Date of filing: 23.01.2018
(51) Int. Cl.: A61K 38/22, A61P 19/00, A61K 9/08, A61K 47/10, A61K 47/26

(54) **THERAPEUTIC AGENT FOR SHORT STATURE**

(30) Priority: 24.01.2017 JP 2017010736; 28.04.2017 JP 2017089563
(71) Applicant: Daiichi Sankyo Company, Limited, Tokyo 103-8426 (JP)
(72) Inventor: MOROZUMI Naomi, Chuo-ku, Tokyo 1038426 (JP); FURUYA Mayumi, Chuo-ku, Tokyo 1038426 (JP); JINDO Toshimasa, Chuo-ku, Tokyo 1038426 (JP); ABE Yasuyuki, Chuo-ku, Tokyo 1038426 (JP); MIEDA Shiuhei, Tokyo 103-8426 (JP); OHNO Akiko, Tokyo 103-8426 (JP)
(74) Representative: Arends, William Gerrit
(86) International application number: PCT/JP2018/002021
(87) International publication number: WO 2018/139464

(57) **Abstract**

The present invention provides a therapeutic agent for failure-to-thrive or short stature which contains C-type natriuretic peptide (CNP) or a CNP derivative as an active ingredient, reduces adverse reactions, and exhibits excellent efficacy, and a method for treating failure-to-thrive or short stature.

## Description

### Technical Field

The present invention relates to a therapeutic agent for failure-to-thrive or short stature which contains C-type natriuretic peptide (CNP) or a CNP derivative as an active ingredient, which reduces adverse reactions, and exhibits excellent efficacy, and a method for treating failure-to-thrive or short stature.

### Background Art

The term "short stature" is generally defined as a height of average height -2 SD or less or a 2-year growth velocity of -1.5 SD or less compared with the heights of a population of the same age, sex and race (HP of The Japanese Society for Pediatric Endocrinology, http://jspe.umin.jp/public/teisinchou.html). "Short stature" develops due to diverse causes such as living environments, stress, heredity, background diseases and abnormalities. Also, it is often difficult to identify its cause. A child's bones have cartilage tissues called growth plates at both ends. The growth plates are thickened by the growth and hypertrophy of chondrocytes or the production of extracellular matrix and then they ossify so that the bones grow. When the growth plates close after puberty, the bones also stop growing. Therefore, it is probably desirable to start the treatment of short stature as early as possible.

For example, growth hormone (GH), insulin-like growth factor-1 (IGF-1), or thyroid hormone are currently used in the treatment of short stature. However, a low proportion of patients respond to this treatment, and no drug is effective for many cases.

Under these circumstances, NPR-B agonists typified by C-type natriuretic peptide (hereinafter, referred to as CNP) and CNP derivatives have been expected as new therapeutic agents for short stature in recent years (Patent Literature 1, Non Patent Literature 1, etc.). CNP specifically binds to NPR-B containing guanylate cyclase and exerts various physiological activities by adjusting intracellular cGMP levels. CNP is considered to participate in the differentiation and the phenotype-expression of chondrocytes in growth plates (Non Patent Literature 2), and has also been found evidently to increase the bone lengths of liver-specific CNP-expressing transgenic mice (Non Patent Literature 3) and normal mice given intravenously administered CNP-22 continuously (Non Patent Literature 4). In Non Patent Literature 4, however, the effective concentration of CNP-22 in plasma was 29.3 ng/mL, and the blood pressure of the animals was not monitored. In Patent Literature 1, mice caused to overexpress CNP in a cartilage-specific manner were prepared using a mouse procollagen a1 type II (Col2a1) promoter region, and a growth promoting effect was confirmed. However, CNP expression levels in chondrocytes continued to increase from birth. Thus, neither can the optimum dosage and administration of CNP as a therapeutic agent be estimated nor can the time required for treatment be identified.

Meanwhile, natriuretic peptide is effective for lowering blood pressure by cardiovascular action. Therefore, the risk of hypotension elevates with the elevation of its concentration in plasma. Hence, for the use of CNP as a therapeutic agent for short stature, it is necessary to set its dosage and administration with consideration for safety (Non Patent Literature 5). In fact, clinical trials on the treatment of achondroplasia with a CNP derivative have reported that the CNP derivative exhibits a significant growth promoting effect by repeated subcutaneous administration to achondroplasia patients once a day for 6 months to 12 months. However, a decrease in blood pressure and a compensatory increase in heart rate have been reported as adverse reactions caused by administration of the drug. Due to insufficient separation of efficacy from adverse reactions, there is apprehension that the dose is not sufficiently increased due to concerns over decreased blood pressure, or that the pharmacological effect is insufficient owing to nonconformity to a dosing schedule.

### Citation List

### Patent Literature

Patent Literature 1: Japanese Patent Laid-Open No. 2003-104908

### Non Patent Literature

Non Patent Literature 1: Yasoda A, et al., Endocr J. (2010), Vol. 57, No. 8, p. 659-666
Non Patent Literature 2: Yasoda A et al., Nat Med. (2004), Vol. 10, No. 1, p. 80-86
Non Patent Literature 3: Kake T et al., Am J Physiol Endocrinol Metab. (2009), Vol. 297, No. 6, p. E1339-E1348
Non Patent Literature 4: Yasoda A et al., Endocrinology (2009), Vol. 150, No. 7, p. 3138-3144
Non Patent Literature 5: Wendt DJ et al., J Pharmacol Exp Ther. 2015, Vol. 353, No. 1, p. 132-49

### Summary of Invention

### Technical Problem

An object of the present invention is to provide a drug that reduces the risk of adverse reactions while maintaining sufficient efficacy when a NPR-B agonist CNP or CNP derivative is used as a therapeutic agent for short stature.

### Solution to Problem

The present inventors have found that a CNP derivative (A) or (B) exhibits a much stronger bone growth effect under continuous subcutaneous administration conditions than that under repeated subcutaneous administration conditions, when administered at the same dose (effective dose) to normal rats. In this test, the present inventors have further successfully detected significant increase in body length and lower limb bone length by medication for a period as very short as 1 week of the continuous subcutaneous administration. The present inventors have also shown that the bone growth effect can be sufficiently separated from a blood pressure lowering effect as an adverse reaction by control of the change in concentration in the plasma, and have actually identified a concentration range in plasma capable of inducing strong bone growth without causing decreased blood pressure.

On the basis of these findings, the present inventors have conducted further studies, reaching the completion of the present invention. The present invention encompasses the following aspects:
(1) A therapeutic agent for failure-to-thrive, comprising CNP or a derivative thereof as an active ingredient, wherein the therapeutic agent, when administered to a human, is controlled such that the concentration in plasma of the active ingredient is kept in the range of 0.1 to 10 ng/mL for 8 hours or longer per day.
(2) The therapeutic agent according to (1), wherein the CNP or the derivative thereof is a peptide consisting of the amino acid sequence of SEQ ID NO: 3 or 4.
(3) The therapeutic agent according to (1), wherein the failure-to-thrive is any member selected from the group consisting of achondroplasia, hypochondroplasia, thanatophoric dysplasia, osteochondrodysplasia, Turner's syndrome, abnormal bone growth induced by steroid therapy, RAS/MAPK syndrome, growth hormone deficiency, growth hormone insensitivity syndrome, thyroid hormone deficiency, chronic renal failure, SHOX gene abnormality, neurofibromatosis type I, SGA short stature, mucopolysaccharidosis, and idiopathic short stature.
(4) The therapeutic agent according to (1), wherein the concentration in plasma of the active ingredient is kept in the range of 0.1 to 4 ng/mL.
(5) The therapeutic agent according to (1), wherein the duration of the concentration in plasma of the active ingredient per day is 16 hours or longer.
(6) The therapeutic agent according to (1), wherein the therapeutic agent is in any form selected from the group consisting of an injection for drip infusion, a controlled-release microcapsule preparation, a controlled-absorption patch preparation for percutaneous administration, a gelled preparation, an isoelectric precipitation preparation, a complex formation preparation and a controlled-release subcutaneous injection preparation (SC infusion pump).
(7) The therapeutic agent according to (6), wherein in the controlled-release subcutaneous injection preparation, the drug solution is a solution containing a CNP derivative consisting of the amino acid sequence of SEQ ID NO: 3 or 4, sucrose, and metacresol. In this context, when the drug solution is provided in the form of a freeze-dried preparation, a kit product of a freeze-dried vial of the active ingredient and sucrose and a dissolving solution of a metacresol solution may be adopted.

### Advantageous Effects of Invention

CNP and a derivative thereof are capable of alleviating and/or improving failure-to-thrive in various diseases and treating short stature resulting from the failure-to-thrive. The expected values of growth velocities as a therapeutic effect differ among patients because of varying causes of failure-to-thrive or short stature and patient backgrounds such as the ages of patients, the presence or absence of concomitant drugs, and the target duration of treatment. The continuous administration of the drug expands a dose range that permits safe treatment and also enhances the therapeutic effect per unit dose. As a result, drastic improvement in QOL of patients is expected. In addition, a therapeutic effect appears very early as compared with intermittent treatment. This can also contribute to improvement in the patient's adherence.

### Brief Description of Drawings

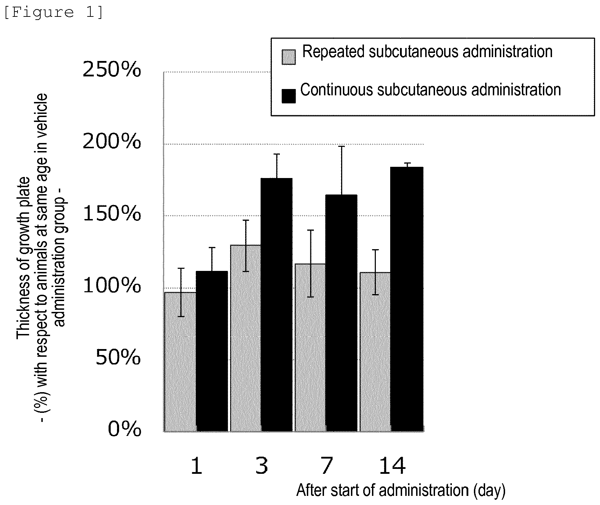

[Figure 1] Figure 1 is a graph showing the thickness of a growth plate at the proximal epiphysis of the tibia when a CNP derivative (A) was continuously subcutaneously administered at a dose of 1 mg/kg/day from 1 to 14 days or repeatedly subcutaneously administered at this dose once a day from 1 to 14 days to 7-week-old male Crl:CD (SD) rats (n = 3 per point in time).

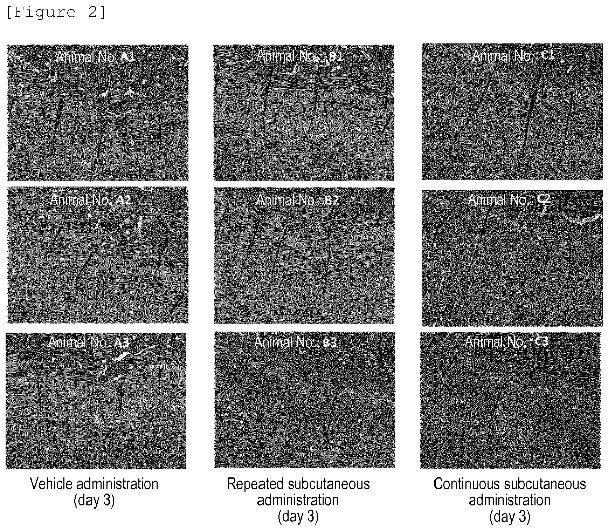

[Figure 2] Figure 2 is a HE-stained specimen image taken near a growth plate at the proximal epiphysis of the tibia when the CNP derivative (A) was continuously subcutaneously administered at a dose of 1 mg/kg/day for 3 days or repeatedly subcutaneously administered at this dose once a day for 3 days to 7-week-old male Crl:CD (SD) rats (n = 3 per point in time).

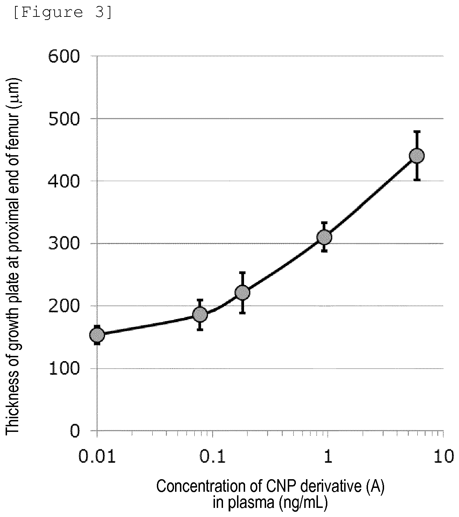

[Figure 3] Figure 3 is a graph showing the thickness of a growth plate at the proximal epiphysis of the femur and the concentration of the CNP derivative (A) in plasma after continuous subcutaneous administration of the CNP derivative (A) at doses of 0.005 to 0.5 mg/kg/day for 7 days to 7-week-old male Crl:CD (SD) rats (n = 5). Data with "*" or "**" indicates that the thickness of the growth plate at the proximal epiphysis of the femur significantly differed from that of a vehicle administration group (p < 0.05 or p < 0.01, an actually measured value was converted to Log and then analyzed by Dunnett's method).

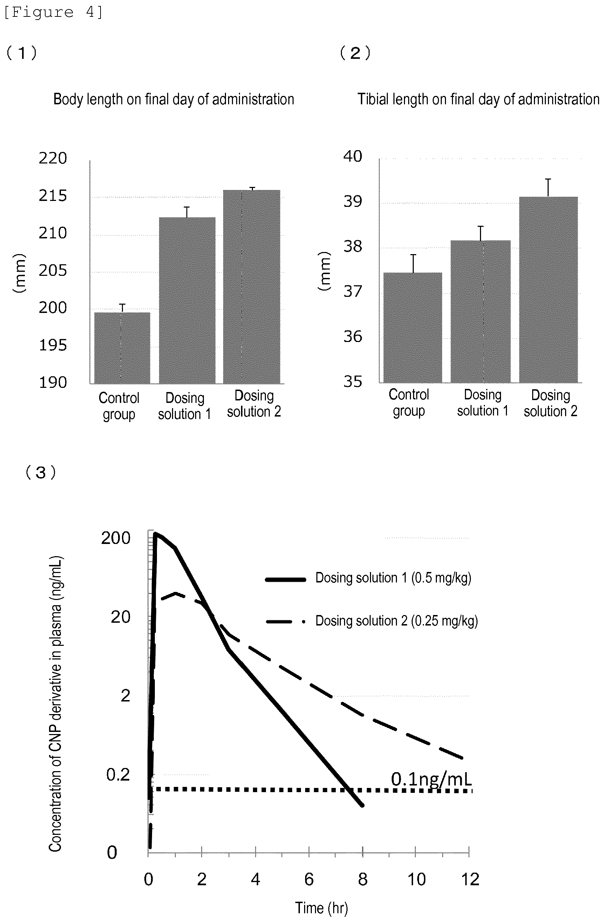

[Figure 4] Figure 4 is a graph showing (1) body length (from the mouth to the end of the tail), (2) lower limb tibial length, and (3) change in concentration of the CNP derivative (A) in plasma under the same conditions (estimated value) at the completion of administration when the CNP derivative (A) was dissolved in two types of vehicles differing in absorption rate from an administration site (vehicle 1: 0.03 mol/L acetate buffer solution (pH 4.0)/1% benzyl alcohol/10% sucrose, vehicle 2: 1% hyaluronic acid solution containing 0.1% L-methionine; solutions of the CNP derivative (A) dissolved in vehicle 1 and vehicle 2 are referred to as dosing solution 1 and dosing solution 2, respectively), and repeatedly subcutaneously administered at a dose of 0.5 mg/0.3 mL/kg or 0.25 mg/0.3 mL/kg once a day for 5 weeks to 7-week-old female Crlj:WI (Wistar) rats (n = 6).

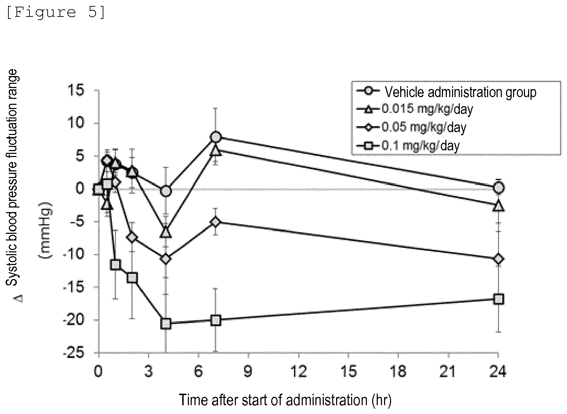

[Figure 5] Figure 5 is a graph showing change (fluctuation range) in systolic blood pressure when the CNP derivative (A) was continuously intravenously administered at doses of 0.015 to 0.1 mg/kg/day at a constant rate for 24 hours to cynomolgus monkeys in which a telemetry transmitter was inserted in advance (two males and two females).

### Description of Embodiments

Hereinafter, the present invention will be described in detail.

The present invention provides a therapeutic agent for failure-to-thrive, comprising a NPR-B agonist (particularly, CNP or a derivative thereof) as an active ingredient, wherein the therapeutic agent, when administered to a human, is controlled such that the concentration in plasma of the active ingredient is kept in the range of 0.1 to 10 ng/mL for 8 hours or longer per day, and a method for treating failure-to-thrive.

### <NPR-B agonist, CNP, and CNP derivative>

In the present invention, the term "natriuretic peptide receptor-B agonist" is used to mean a substance having an effect of binding to natriuretic peptide receptor-B NPR-B (also called guanylate cyclase B (GC-B); hereinafter, also simply referred to as "NPR-B") to activate its guanylate cyclase (hereinafter, referred to as "NPR-B agonistic activity"), and is also simply referred to as a "NPR-B agonist" in the present specification. Typical examples of the NPR-B agonist include C-type natriuretic peptide (CNP). The NPR-B agonist of the present invention is not particularly limited as long as the NPR-B agonist is a substance having NPR-B agonistic activity. For example, CNP, an active fragment thereof and their mutants, their derivatives and their modified forms can be used. Even a peptide or a low-molecular weight compound lacking structural commonality with CNP is also included in the agonist of the present invention as long as the peptide or the compound has NPR-B agonistic activity.

Examples of the CNP according to the present invention include human-derived CNP-22 composed of 22 amino acids (hCNP-22: GLSKGCFGLK LDRIGSMSGL GC: SEQ ID NO: 1, which has a common amino acid sequence in mammals such as pigs and rats), and human-derived CNP-53 (hCNP-53, amino acid sequence: DLRVDTKSRA AWARLLQEHP NARKYKGANK KGLSKGCFGL KLDRIGSMSG LGC: SEQ ID NO: 2).

In each CNP, a ring structure formed through a disulfide bond between two C residues contained in the sequence (e.g., a ring structure formed through a disulfide bond between C at the 6-position and C at the 22-position of SEQ ID NO: 1 in hCNP-22) is considered to be important for binding to the NPR-B receptor and the exertion of activity (Furuya M et al., Biochem. Biophys. Res. Commun. (1992), Vol. 183, No. 3, p. 964-969; Silver MA, Curr. Opin. Nephrol. Hypertens. (2006), Vol. 15, p. 14-21; and Calderone A, Minerva Endocrinol. (2004), Vol. 29, p. 113-127). The report of Furuya et al. discloses that, for example: CNP6-22 which is a peptide consisting of the ring structure (peptide consisting of amino acid positions 6 to 22 of SEQ ID NO: 1), or a peptide prepared by adding the N-terminal and C-terminal sequences of the ring structure of ANP to the N terminus and the C terminus, respectively, of the ring structure also exhibits NPR-B agonistic activity almost equivalent to that of hCNP-22; and the activity of a peptide having mutations in L at the 9-position and K at the 10-position of hCNP-22 is attenuated, whereas a peptide having mutations at the other sites (e.g., S at the 16-position and M at the 17-position) or a peptide prepared by substituting an amino acid sequence from the 10- to 12-positions of ANP by a corresponding hCNP-22 sequence L-K-L (9- to 11-positions of SEQ ID NO: 1) exhibits NPR-B agonistic activity almost equivalent to that of hCNP-22.

In the present invention, the term "active fragment" of a peptide or a protein having biological activity is used to mean a substance that consists of a site related to the biological activity of the peptide or the protein and retains at least a portion of the biological activity of the peptide or the protein. A peptide that consists of at least a partial amino acid sequence of the amino acid sequence of SEQ ID NO: 1 or 2 and has NPR-B agonistic activity can be used as the active fragment of CNP according to the present invention.

The aforementioned active fragment itself may be adopted as the NPR-B agonist of the present invention. Also, even a peptide derived from the active fragment by the addition of one or more amino acids to the N terminus or the C terminus, or both (derivative of the active fragment) can be adopted as long as the desired agonistic activity is retained.

In the present invention, the term "mutant" of a peptide or a protein having biological activity is used to mean a substance that has an amino acid sequence derived from the amino acid sequence of the peptide or the protein by the substitution, deletion, insertion, and/or addition (hereinafter, referred to as "substitution, etc.") of one to several amino acids at one to several sites, and retains at least a portion of the biological activity of the peptide or the protein. The "several sites" are usually 3 sites, and preferably 2 sites. The "several" amino acids are usually 10 amino acids, preferably 5 amino acids, more preferably 3 amino acids, and further preferably 2 amino acids. The substitution, etc. at several sites may be any one of substitution, deletion, insertion and addition or may be a combination of two or more thereof. The amino acids to be substituted, etc. may be naturally occurring amino acids, may be modified forms thereof such as acylated forms, or may be artificially synthesized amino acid analogs. The sites of substitution, etc. may be selected from any site as long as a portion of the activity of the original peptide or protein is retained. A site other than the active site or the receptor binding site of the original peptide or protein is preferably substituted, etc.

For example, a mutant having substitution, etc. at the desired site can be adopted as the mutant of CNP as long as NPR-B agonistic activity is retained. Preferred examples thereof can include a peptide that retains the aforementioned ring structure sequence and has substitution, etc. at any other site. Specifically, the mutant of CNP may have the substitution, etc. of one to several amino acids at one or more desired sites in the amino acid sequence shown in SEQ ID NO: 1 or 2 as long as the mutant has NPR-B agonistic activity. The mutant of CNP is preferably a mutant having the substitution, etc. of one to several amino acids at one to several sites of amino acids other than the amino acids shown in SEQ ID NO: 5 in the amino acid sequence of SEQ ID NO: 1 or 2, and more preferably a mutant having the substitution, etc. of one to several amino acids at any one to several sites selected from the 1- to 5-positions of the amino acid sequence shown in SEQ ID NO: 1, or a mutant having the substitution, etc. of one to several amino acids at any one to several sites selected from the 1- to 31-positions of the amino acid sequence shown in SEQ ID NO: 2.

The aforementioned mutant itself may be adopted as the NPR-B agonist of the present invention. Also, even a peptide derived from the mutant by the addition of one or more amino acids to the N terminus or the C terminus, or both (derivative of the mutant) can be adopted as long as the desired agonistic activity is retained.

As specific examples of the mutant of CNP, it has been reported that, for example: a peptide having mutations at the 17- and 18-positions of hCNP-22 has the same NPR-B agonistic activity as that of hCNP-22; a mutant derivative prepared by replacing the N terminus and the C terminus of the ring structure moiety of such a mutant with ANP-derived sequences also exhibits NPR-B agonistic activity of about 90% of the activity of hCNP-22; a peptide having a mutation at any one of the 9- to 11-positions exhibits NPR-B agonistic activity of 50% or more of the activity of hCNP-22; and a peptide having mutations at both the 10- and 11-positions has NPR-B agonistic activity of 40% or more of the activity of hCNP-22 (Furuya M et al., Biochem. Biophys. Res. Commun., (1992), Vol. 183, No. 3, p. 964-969). Other literature states that, for example: various mutants of hCNP-22 retain NPR-B agonistic activity; and some of these mutants also have resistance to cleavage by neutral endopeptidase (NEP) which is a CNP degrading enzyme (WO2009/067639).

In the present invention, the term "derivative" of a peptide or a protein having biological activity is used to mean a fusion peptide comprising the amino acid sequence of the peptide or the protein and further comprising another peptide or protein added thereto, the fusion peptide retaining at least a portion of the biological activity of the physiologically active peptide or protein. Such a fusion peptide having at least a portion of the biological activity (in the present invention, the effect of binding to NPR-B to activate its guanylate cyclase) is also referred to as a derivative of the physiologically active peptide. In the derivative of the present invention, the added peptide may be fused with one of the C terminus and the N terminus of the original physiologically active peptide or protein, or the added peptide may be fused with both the C terminus and the N terminus. The peptide to be added is not particularly limited, and the peptide itself preferably lacks physiological activity. The added peptide may be bound directly or may be bound via a linker sequence composed of one to several amino acids. While various linker sequences are known, a linker sequence rich in G, S, or the like is often used. Examples of such an added peptide can include the Fc sites of immunoglobulins (preferably IgG), serum albumin, and the C-terminal partial sequences of ghrelin. Examples of the derivative for use as the NPR-B agonist of the present invention can include derivatives of CNP (preferably hCNP-22 or hCNP-53), and derivatives of active fragments (preferably hCNP6-22) of CNP. A derivative of hCNP-22 or a derivative of hCNP6-22 is preferred.

Specific examples of the derivative of CNP can include various CNP derivatives disclosed in U.S. Patent Publication No. US2010-305031 (corresponding International Publication No. WO2009/142307) (SEQ ID NOs: 109, 110, 124 to 130, 157 and 158 in the patent literature of this U.S. patent publication). This literature has reported that a derivative prepared by adding a partial sequence derived from the C-terminal side of ghrelin to a physiologically active peptide such as ANP, CNP, or motilin exhibited improved retention in blood while retaining the physiological activity of the original peptide. In this report, all of a range of derivatives prepared by adding a peptide comprising Wk-Xl-Y-Zm-Wn (wherein W is a basic amino acid such as Lys or Arg; Y is an acidic amino acid such as Asp or Glu; X and Z may be the same or different and may each be any amino acid except for acidic amino acids and basic amino acids; k and n each independently represent an integer of 1 or 2; 1 and m each independently represent a natural number of 0, 1 or 2; and examples of such a sequence preferably include RKESKK, RKDSKK, RKSEKK, and RKSDKK) derived from the C terminus of ghrelin to any one of the N terminus and the C terminus, or both, of CNP, retained NPR-B agonistic activity and had a prolonged half-life in blood. A CNP derivative (A) (SEQ ID NO: 3) used in the Examples of the present invention is a typical example of the CNP derivatives prepared in the patent literature. The description of the patent literature is incorporated herein in its entirety.

As for the derivative of CNP or the active fragment thereof, a peptide prepared by adding the C-terminal moiety of ANP to the C terminus of hCNP-22, or a peptide prepared by adding the N-terminal moiety and the C-terminal moiety of ANP to the N terminus and the C terminus of hCNP6-22 (derivative of the active fragment of CNP) also retained NPR-B agonistic activity equivalent to that of hCNP-22 (Furuya M et al., Biochem. Biophys. Res. Commun., (1992), Vol. 183, No. 3, p. 964-969). Other literature states that, for example: various derivatives of hCNP-22 and hCNP-53 retain NPR-B agonistic activity; and among them, a plurality of derivatives further have NEP degradation resistance (WO2009/067639). Among the derivatives described in the patent literature of this international publication, a derivative consisting of the amino acid sequence Pro-Gly-CNP-37 (SEQ ID NO: 4), which is defined as a CNP derivative (B) in the present specification, has been reported to have a pharmacological effect on achondroplasia model mice (Florence L et al., Am. J. Hum. Genet., (2012), Vol. 91, No. 6, p. 1108-1114, body copy: http://www.sciencedirect.com/science/article/pii/S0002929 71200537X: Supplement::http://download.cell.com/AJHG/mmcs/journals/0 002-9297/PIIS000292971200537X.mmc1.pdf).

Further examples of the derivative of CNP-22 of the present invention can include a peptide consisting of an amino acid sequence derived from the amino acid sequence (SEQ ID NO: 2) of human CNP-53 by the deletion of 1 or more and 30 or fewer consecutive amino acids from the N terminus, specifically, CNP-36 (amino acid positions 18 to 53 of SEQ ID NO: 2). Such a derivative is preferably a peptide consisting of an amino acid sequence derived from the amino acid sequence of hCNP-53 (SEQ ID NO: 2) by the deletion of 10, 11, 12, 13, 14, 15, 16, 17, 18, 19, 20, 21, 22, 23, 24 or 25 consecutive amino acids from the N terminus thereof, and more preferably hCNP-36 (peptide consisting of an amino acid sequence from amino acid positions 18 to 53 of SEQ ID NO: 2) derived from hCNP-53 by the deletion of 17 consecutive amino acids from the N terminus thereof.

These various CNP derivatives and derivatives of the active fragment of CNP can preferably be used as the NPR-B agonist of the present invention. The derivative is more preferably a derivative consisting of an amino acid sequence derived from SEQ ID NO: 2 by the deletion of 1 or more and 30 or fewer (preferably 25 or fewer, and more preferably 20 or fewer) consecutive amino acids from the N terminus, or a derivative of SEQ ID NO: 3 or 4, and further preferably a CNP derivative (A) (SEQ ID NO: 3) or a CNP derivative (B) (SEQ ID NO: 4).

In the present invention, the term "modified form" of a peptide or a protein having biological activity is used to mean a substance that has a modification at one to several sites of amino acids contained in the peptide or the protein through chemical reaction with another chemical substance, and retains at least a portion of the biological activity of the peptide or the protein. The site to receive the modification may be selected from any site as long as the activity of the original peptide or protein is retained. For example, for a modification to add a somewhat large chemical substance such as a polymer, it is preferred to modify a site other than the active site or the receptor binding site of the peptide or the protein. In the case of a modification for preventing cleavage by a degrading enzyme, it is preferred to modify a site that is subject to the cleavage.

For example, the modified form of CNP may have the modification at one or more desired sites in the amino acid sequence of SEQ ID NO: 1 or 2 as long as the modified form has NPR-B agonistic activity. The modified form preferably has the modification at one to several sites of amino acids other than the amino acids shown in SEQ ID NO: 5 in the amino acid sequence of SEQ ID NO: 1 or 2, and more preferably has the modification at any one to several sites selected from the 1- to 5-positions of the amino acid sequence of SEQ ID NO: 1. In the case of a modification to confer resistance to cleavage by NEP, which is known to cause cleavage between C at the 1-position and F at the 2-position of SEQ ID NO: 5 in ring structures contained in various CNP peptides, this bond may be modified.

Modified forms of the active fragment of CNP, the mutant and their derivatives mentioned above are also included in the present invention. Such various modified forms can also be used in the present invention as long as NPR-B agonistic activity is retained.

Various methods are known as methods for the chemical modification. For example, a method of adding high-molecular polymer, such as polyethylene glycol (PEG) or polyvinyl alcohol (PVA), which is used in pharmaceutical techniques (pharmacologically used), or a method of adding a compound serving as a linker to the side chain amino group of a K residue or the like and binding thereto another protein or the like (e.g., serum albumin) via this linker, is known. Various methods can be adopted without limitations thereto.

As specific examples of such modified forms of CNP, the active fragment thereof, their mutants and their derivatives, for example, WO2009/067639 discloses that: modified forms in which various hydrophilic polymers such as PEG are bound to hCNP-22 and hCNP-53, or a plurality of modified forms prepared by replacing a Cys6-Phe7 peptide bond which is a site of cleavage by NEP in hCNP-22 with a pseudopeptide bond such as -CH₂-NH- or -C(=O)-N(R)- (wherein R is a lower alkyl group such as a methyl group, an ethyl group, a propyl group, an isopropyl group, a n-butyl group, an isobutyl group, a sec-butyl group, or a tert-butyl group) retain NPR-B agonistic activity; and many of them have improved retention in blood as compared with hCNP-22. As for methods for producing modified forms of various physiologically active peptides, these modified forms can be appropriately prepared with reference to, for example, U.S. Patent Publication No. US2009-0175821.

The ring structure is important for the binding of CNP to a NPR-B receptor. Therefore, a derivative or a modified form with another sequence or substance bound to the terminal moiety is, in particular, expected to a sufficient degree to retain NPR-B agonistic activity without inhibiting binding to the NPR-B receptor, because the added peptide or the modifying substance has less influence on the ring structure. This has been demonstrated by many of the items in the literature mentioned above.

CNP, the active fragment thereof, their mutants, their derivatives and their modified forms described above may be collected from natural cells or tissues, may be produced by use of a genetic engineering or cellular engineering approach, may be chemically synthesized, or may undergo the modification of an amino acid residue or the removal of a partial amino acid sequence by the enzymatic treatment or chemical treatment thereof. Such a substance can be appropriately produced according to a common method with reference to the description of the present specification or the cited literature.

A person skilled in the art can readily determine whether or not a certain substance has NPR-B agonistic activity, by a heretofore known method. Specifically, this is attained by adding the substance to cultured cells forced to express NPR-B (Suga S et al., Endocrinology (1992), Vol. 130, No. 1, p. 229-239), and measuring the intracellular cGMP level. The phrase "a portion of NPR-B agonistic activity is retained" is used to mean that in the case of arranging a NPR-B agonist substance and CNP using the same test system and measuring NPR-B agonistic activity, the peak of cGMP elevating activity retains at least 10% or more, preferably 30% or more, more preferably 50% or more, and further preferably 70% or more, of the peak of cGMP elevating activity exhibited by CNP. Even a substance that does not exhibit the large elevation of activity in the peak can be used in the present invention as long as the substance exhibits favorable results in relation to activity duration and retention in blood when administered to an organism.

Even a compound (e.g., low-molecular weight compound) lacking a structure common to natriuretic peptide can also be used as the NPR-B agonist in the present invention as long as the compound improves the ability to produce cGMP by the addition of the low-molecular weight compound to such an evaluation system.

The NPR-B agonist of the present invention is preferably a CNP derivative, more preferably hCNP-53, a CNP derivative (A): CNP (1-22) ghrelin (12-28, E17D, SEQ ID NO: 3), or a CNP derivative (B): Pro-Gly-CNP-37 (SEQ ID NO: 4), and further preferably a CNP derivative (A) or a CNP derivative (B). The hCNP-53 and the CNP derivatives (A) and (B) are resistant to metabolism by neutral endopeptidase which is the main metabolic enzyme of natriuretic peptide, and have the property of long persistence *in vivo,* as compared with CNP-22. In addition, sites other than the ring structure serving as a site responsible for the NPR-B receptor agonistic activity of CNP are rich in basic amino acids, and penetration into cartilage tissues and retention after penetration are improved. Hence, their concentrations in cartilage tissues are easily kept high as compared with the concentrations in plasma. Thus, an excellent bone growth effect is probably exhibited at the concentration range in plasma, which is a feature of the present invention, *in vivo.*

### <Recipient>

The NPR-B agonist of the present invention can be used as an active ingredient in a medicament for alleviating and/or improving failure-to-thrive in a growing individual having short stature or predicted to manifest short stature in the future due to failure-to-thrive, by administration thereof to the subject.

The recipient of the medicament of the present invention is not particularly limited as long as the recipient is a mammal. The recipient is, for example, a human, a monkey, a dog, a cat, or a horse and is preferably a human. In the present invention, the term "growing" is used to mean a phase in which growth plates are maintained without closure of the epiphysial lines of the long bones of the subject. In the case of humans, the growing phase differs among individuals, but is usually age 18 or lower, preferably age 15 or lower, and more preferably age 12 or lower. The average body weight of the subject in this period is presumed to be 3.0 to 66 kg. The medicament of the present invention alleviates and/or improves failure-to-thrive and is therefore much more likely to produce its effect by administration to the subject in a phase in which the growth velocity is fast.

The medicament of the present invention is also capable of treating short stature resulting from failure-to-thrive. The term "short stature" is generally defined as a height of average height -2 SD or less or a 2-year growth velocity of -1.5 SD or less compared with the heights of a population of the same age, sex and race. In this case, the medicament of the present invention is administered in a range in which the subject is in the growing phase and usually has a height that does not exceed average height + 2 SD of a population at the same age as that of the subject.

In the present invention, the term "alleviate" as applied to the failure-to-thrive is used to mean that at least one or more of the effects of, for example, preventing the occurrence of the disorder, relieving or delaying the progression of the disorder, and reducing the degree of the disorder are exerted so that short stature that may result from failure-to-thrive does not occur, or a course expected to relieve the degree of short stature is taken.

In the present invention, the term "improve" as applied to the failure-to-thrive is used to mean that at least one or more of the effects of, for example, improving the rate of growth of the recipient to a level equal to or greater than the average rate of growth of a population at the same age, preventing short stature in the course of progression of failure-to-thrive, recovering from the resulting short stature, and bringing the resulting disorder state close to a normal state at the completion of an event inducing the disorder are exerted so that the height of the subject is closer to the average height range of individuals at the same age.

The medicament of the present invention is not particularly limited as long as the medicament contains the NPR-B agonist of the present invention as an active ingredient and is prescribed for the purpose of alleviating and/or improving failure-to-thrive or treating short stature resulting from failure-to-thrive, in a growing subject having short stature or predicted to manifest short stature in the future due to failure-to-thrive. It is preferred that a package insert of the medicament should state that, for example, the medicament of the present invention is administered for the purpose of alleviating and/or improving failure-to-thrive or treating short stature resulting from failure-to-thrive.

Failure-to-thrive occurs due to various causes. The medicament of the present invention is capable of alleviating or improving failure-to-thrive, regardless of the causes. Such a cause may be failure-to-thrive caused by a disease, for example, achondroplasia, hypochondroplasia, chondrodysplasia, Turner's syndrome, RAS/MAPK syndrome, growth hormone deficiency, growth hormone insensitivity syndrome, thyroid hormone deficiency, chronic renal failure, SHOX gene abnormality, neurofibromatosis type I, SGA short stature, or mucopolysaccharidosis, or may be failure-to-thrive such as abnormal bone growth induced by treatment with a drug such as a steroid.

The failure-to-thrive caused by a disease is diagnosed by clinical findings characteristic of each disease and/or genetic testing, etc. When a subject is diagnosed as having such a disease that causes failure-to-thrive, it is preferred to administer the drug of the present invention thereto even if the subject manifests neither a marked short height nor a phenotype of failure-to-thrive. For example, achondroplasia is reportedly caused by a mutation in the FGFR3 gene. This disease often exhibits phenotypes such as a characteristic face, short extremities, and brachydactyly and is often diagnosed early after birth on the basis of these findings. The CNP derivative (B) used in the Examples of the present specification is under clinical development with achondroplasia as an indication.

### <Approach of keeping concentration in plasma of active ingredient of medicament of present invention>

The medicament of the present invention relates to a preparation form that keeps a given concentration range for a given time or longer when administered to a human, and is thereby capable of reducing the risk of hypotension symptoms or an increased heart rate resulting from the vasodilating effect or blood pressure lowering effect of the NPR-B agonist, and alleviating and/or improving failure-to-thrive or treating short stature resulting from failure-to-thrive, in the recipient.

The substance that can be used as the active ingredient in the medicament according to the present invention may be a free form of the aforementioned NPR-B agonist (CNP or derivative thereof) or may be a pharmaceutically acceptable salt thereof. Specifically, in the present invention, an acid-addition salt of the aforementioned NPR-B agonist with an inorganic acid, for example, hydrochloric acid, sulfuric acid, or phosphoric acid, or an organic acid, for example, formic acid, acetic acid, butyric acid, succinic acid, or citric acid may be used as the active ingredient. Alternatively, in the present invention, a metal salt of the aforementioned NPR-B agonist with sodium, potassium, lithium, calcium, or the like, or a salt form thereof with an organic base may be used as the active ingredient. The pharmaceutical composition according to the present invention may contain a free form of the substance related to the active ingredient, or a pharmaceutically acceptable salt thereof.

The pharmaceutical composition of the present invention comprises the NPR-B agonist or the pharmaceutically acceptable salt thereof as an active ingredient and is prepared by further using a desired carrier or excipient, other additives, diluents, and the like usually used for formulations. Examples of the pharmaceutical carrier or excipient can include hyaluronic acid, chondroitin sulfate, lactose, magnesium stearate, starch, talc, gelatin, agar, pectin, gum arabic, olive oil, sesame oil, cacao butter, ethylene glycol, and other carriers or excipients commonly used.

An injection includes an aseptic aqueous or nonaqueous solution, a suspension, and an emulsion. The aqueous solution and suspension contain, for example, injectable water or an injectable normal saline solution. The nonaqueous solution and suspension contain, for example, propylene glycol, polyethylene glycol, a plant oil such as olive oil, an alcohol such as ethanol, or Polysorbate 80(R). Such a composition may further contain an adjuvant such as an antiseptic, a wetting agent, an emulsifier, a dispersant, a stabilizer (e.g., lactose), or a solubilizing agent (e.g., glutamic acid and aspartic acid). These can be rendered aseptic by a usual sterilization method, for example, filter sterilization through a microfiltration membrane, heat sterilization such as high-pressure steam sterilization, or blending of a bactericide. The injection may be a solution preparation or may be freeze-dried for dissolution and reconstitution before use. For example, a sugar alcohol or a sugar, such as mannitol or glucose can be used as an excipient for the freeze drying.

In the present invention, the active ingredient keeps a given concentration range in plasma for a given time or longer when administered to a human. The upper limit of the concentration range in plasma is usually about 10 ng/mL or lower, preferably about 8 ng/mL or lower, more preferably about 6 ng/mL or lower, and further more preferably about 4 ng/mL or lower. The lower limit of the concentration range in plasma is usually about 0.1 ng/mL or higher, preferably about 0.2 ng/mL or higher, more preferably about 0.3 ng/mL or higher, and further more preferably about 0.4 ng/mL or higher. The duration of the concentration in plasma described above is usually 8 hours or longer per day, and preferably 10 hours or longer, 12 hours or longer, 16 hours or longer, or 18 hours or longer per day. The frequency of administration (drug replacement) differs depending on the adopted preparation form and is usually twice or less a day, and a frequency of administration (replacement) such as, once a day, once per a plurality of days, once a week, or once a month can be adopted.

In the present invention, the term "keep" as applied to the concentration range in plasma of the active ingredient is used to mean that the concentration in plasma is continuously sustained in the predetermined range for the predetermined time or longer. For example, when transient elevation in concentration in plasma (initial burst) immediately after administration is observed as seen in a local sustained-release preparation or the like, the time during which a concentration exceeding the predetermined range is exhibited due to the initial burst is excluded from the duration. It is necessary to design the preparation such that the duration of the concentration in plasma being within the range is the predetermined time or longer. Furthermore, such rapid elevation of the active ingredient concentration as is ascribable to the initial burst has the risk of developing hypotension. Therefore, it is preferred to design the preparation so as to minimize the upper limit of the elevation of the concentration or to exhibit a gradual elevation of the concentration.

Various methods can be applied to a method for keeping the concentration in plasma of the active ingredient in the medicament of the present invention.

For example, controlled release is attained by a pharmaceutical technique of encapsulating the active ingredient in a biodegradable polymer such as polylactic acid, a lactic acid/glycolic acid copolymer, polycaprolactam, or polyphosphazene, and forming a particle or rod shape. In this case, the desired concentration in plasma can be kept by adjusting the quantity of the active ingredient to be encapsulated, the type of the polymer and the blending ratio. Such a preparation can be subcutaneously administered to or locally implanted into an affected area (joints of the extremities, etc.). Subcutaneous administration is preferred because long-term administration is necessary.

The rate of absorption or dissolution from an administration site can be reduced by adopting a recipe of the preparation that causes the active ingredient peptide to be gelled, to form precipitates at a local administration site, or to be complexed, for example, by adopting a local administration preparation such as a subcutaneous injection or a percutaneous administration preparation (Priyanka Agarwal et al., Drug Discovery Today (2013) Vol. 18, Nos. 7/8, pp. 337-349).

The concentration range in plasma of interest can also be kept for the predetermined time by adopting injection through intravenous drip infusion or an administration device such as a subcutaneous infusion pump, and setting the desired dosing rate and dosing solution concentration.

Among patch preparations for percutaneous administration, a controlled-absorption patch preparation for percutaneous administration can control the concentration in plasma depending on the amount of the active ingredient charged in the preparation, the recipe thereof, the manner of application to the skin and the area thereof. Such a preparation may be adopted in the present invention.

The structure of the active ingredient itself can be modified with the aim of acquiring resistance to neutral endopeptidase which is a metabolic enzyme of CNP, or suppressing tubular secretion in the kidney. Also, an effective approach is to suppress tubular secretion in the kidney by increasing the rate of binding to proteins and the like in circulating plasma, or to administer a mixture with a substance serving as a carrier. However, structural modification or formulation design that interferes with the penetration of the active ingredient into extravascular tissues and reduces the *in vivo* volume of distribution is not preferred because of the possibility of interfering with the penetration of the active ingredient of the medicament of the present invention into cartilage tissues.

In the case of adopting, for example, a subcutaneous infusion pump, a drug solution is communicated between an infusion set attached to the skin of a patient and a reservoir containing the drug solution, and a cannula at the tip of the infusion set is subcutaneously inserted or placed in the patient to administer the drug solution. A controller which controls the infusion of the drug solution is usually attached to this apparatus. The desired concentration in plasma can be kept by adopting an appropriate infusion rate according to the concentration of the drug solution depending on the setting. A biological reaction sensor may also be attached to such an apparatus (WO2008/086541). Therefore, the apparatus may be programmed such that, for example, the predetermined concentration range is kept by monitoring the concentration of the active ingredient or cGMP in blood, or, when abnormally decreased blood pressure or an abnormal variation in pulse is detected in a patient, the infusion rate is transiently decreased, or infusion is stopped, and the infusion rate is brought back to the original one after detection of the normalization of the blood pressure or the pulse. Such a subcutaneous infusion pump is clinically applied to insulin preparations and is distributed from a plurality of manufacturers such as Medtronic plc (MiniMed(R)) and TOP Corp. (syringe pump). Although the specification of a product differs among manufacturers, the selection of the active ingredient concentration of a drug solution contained in a reservoir, and the setting of an appropriate dosing program are appropriately performed for the desired subcutaneous infusion pump on the basis of the disclosure of the present specification and known techniques in order to achieve the keeping of the desired concentration in plasma. For example, in the case of continuously administering a dose of 3 to 50 µg/kg/day for 24 hours to a growing patient (3 to 66 kg), a drug solution concentration of 0.1 mg/mL to 5.0 mg/mL in the reservoir and an infusion rate of 0.5 to 200 µL/ hr can be adopted for achieving the object of the present invention. The parameters of this dosage and administration, such as the drug solution concentration and the infusion rate, are appropriately adjusted according to the body weight of the recipient, the type of device, and the dosing program (daily infusion time, dosing interval, etc.).

An appropriate combination of additives adopted for typical peptide injections can be adopted as the recipe of the drug solution for such a subcutaneous infusion pump. The solvent is usually water, and a buffer and/or a pH adjuster may be appropriately added thereto according to the need for pH adjustment. A pharmaceutical substance usually used, such as citric acid or histidine can be appropriately adopted as the buffer. The pH of the solution can be appropriately selected and is preferably pH 5.0 to pH 6.0. In the case of adopting a histidine buffer solution, the optimum pH is pH 5.2. The subcutaneous infusion pump needs to administer the same drug solution continuously for several days. Therefore, a stabilizer is important for ensuring stability at normal temperatures. Preferred examples of the stabilizer can include sucrose. Its content can be appropriately selected from about 5 to 30% and is preferably about 10%. Since the administration device is in contact with the human body for a long time, it is anticipated that there may be a risk of contaminating the drug solution. Therefore, it is also important to add a preservative as a measure against any contamination. For example, metacresol can be adopted as the preservative. Its content can be appropriately selected from about 0.001 to 1% (preferably from 0.003 to 0.3%). In the case of adopting a freeze-dried preparation as a preparation form, there is a risk of causing a change in content at the time of administration due to the evaporation of metacresol in the freeze drying step. Therefore, it is preferred to prepare a dosing solution by dissolving a freeze-dried product using an appropriate concentration of a metacresol solution, without having added metacresol to the solution for freeze drying. The present invention also provides such a kit preparation comprising a combination of a freeze-dried product of the active ingredient and a metacresol-containing dissolving solution enclosed in different containers.

The active ingredient of the present invention might undergo photodegradation in solution. Therefore, it is preferred to take measures to avoid exposure to light during storage and during use. For example, an element subjected to light shielding treatment can be adopted in a container or a channel. The keeping of the predetermined concentration in plasma by the active ingredient of the present invention can be confirmed by collecting the blood of the recipient patient over time for a given period, and measuring the content of the active ingredient in plasma. Such confirmation of the concentration in plasma is performed during a clinical trial on the medicament to determine an appropriate dose and administration method. Hence, confirmation for each recipient patient is unnecessary. The predetermined concentration in plasma is achieved according to the dosage and administration determined by the clinical trial.

The pharmaceutical composition according to the present invention may be used concomitantly with an additional bone formation promoter, bone resorption inhibitor or therapeutic agent for short stature. Examples of such a partner drug for concomitant use can include, but is not limited to, bisphosphonate preparations, bone morphogenetic protein (BMP) preparations, parathyroid hormone (PTH) preparations, basic fibroblast growth factor (bFGF) preparations, anti-osteoclast differentiation factor (RANKL) antibody preparations, growth hormone preparations, and insulin-like growth factor (IGF-1) preparations.

### Examples

Hereinafter, the present invention will be described in more detail with reference to Examples. These Examples illustrate one example of the embodiment of the present invention, and the present invention is not limited by these Examples.

The CNP derivative (A) (SEQ ID NO: 3) used in the present Examples was prepared on the basis of the description of U.S. Patent Publication No. US2010-305031. Also, the CNP derivative (B) (SEQ ID NO: 4) was prepared on the basis of the description of WO2009/067639.

### <Example 1> Confirmation of growth plate thickening effect

### [Method]

A vehicle or the CNP derivative (A) was repeatedly subcutaneously administered at a dose of 1 mg/kg/day once a day or continuously subcutaneously administered at this dose using an osmotic pump (MINI-OSMOTIC PUMP (manufactured by DURECT Corp., flow rate: 0.5 µL/hr)), to week-old male Crlj:WI (Wistar) rats. The right lower limb knee parts were collected 1, 3, 7 and 14 days after the start of administration and fixed in a neutral formalin buffer solution, followed by the preparation of HE-stained specimens.

### [Results]

Figure 1 shows the results of comparing the thickness of a growth plate at the proximal end of the tibia after medication with the CNP derivative (A) when the thickness of a growth plate at the proximal end of the right tibia collected from rats of the same age in weeks without the administration of the CNP derivative (A) (vehicle administration group) was used as a reference (100%). Under both the administration conditions, the thickening of the growth plate at the proximal end of the tibia was observed at least 3 days after the start of administration (Figure 2). The degree of thickening was much higher under the continuous subcutaneous administration conditions than under the once-a-day repeated subcutaneous administration conditions.

### <Example 2> Comparison of exertion of bone growth effect between repeated subcutaneous administration conditions and continuous subcutaneous administration conditions.

### [Method]

A dosing solution of the CNP derivative (A) or (B) was repeatedly subcutaneously administered at doses of 0.005, 0.015, 0.05, 0.15 and 0.5 mg/kg/day once a day for 7 days to 7-week-old male Crl:CD (SD) rats, or charged in an osmotic pump (MINI-OSMOTIC PUMP Model 2002, manufactured by DURECT Corp., flow rate: 0.5 µL/hr, for 14-day use), implanted in the backs of rats, and continuously subcutaneously administered at these doses for 7 days. Their body lengths (from the mouth to the end of the tail) were measured before the start of administration and 8 days after the start of administration.

### [Results]

For both the CNP derivatives (A) and (B), the bone growth effect per dose was stronger under continuous subcutaneous administration conditions, and a significant increase in body length (from the mouth to the end of the tail) was observed for the 7-day administration at doses of 0.15 mg/kg/day or higher, whereas no significant effect was observed under repeated subcutaneous administration conditions (Tables 1 and 2). From the individuals given the continuous and subcutaneous administration of CNP derivative (A), plasma was collected at the time of dissection, and the concentration of CNP derivative (A) in the plasma was measured to confirm its relationship with the thickness of the growth plate at the proximal end of the femur obtained by the dissection. The results are shown in Figure 3. Significant thickening of the growth plate at the proximal end of the femur was observed for doses of 0.015 mg/kg/day or higher under continuous subcutaneous administration conditions. In this respect, the concentration of the CNP derivative (A) in the plasma collected at the time of dissection was 0.034 ng/mL, suggesting that a significant bone growth effect is exhibited by keeping the concentration in plasma at at least 0.05 ng/mL or higher.

[Table 1: Body length (from mouth to end of tail) after completion of 7-day repeated subcutaneous administration or 7-day continuous subcutaneous administration of CNP derivative (A) to 7-week-old male SD rat]
(Mean ± standard deviation, n = 4 or 5)

### CNP derivative (A)

**[Table 1]**

| Group | Dose | Body length (from mouth to end of tail) (mm) | | | | | |
|---|---|---|---|---|---|---|---|
| | | Repeated subcutaneous administration | | | Continuous subcutaneous administration | | |
| | (mg/kg/day) | Mean | ± standard deviation | | Mean ± | standard deviation | |
| Group 1 | 0 | 212.4 | ±5.6 | ⁻ | 215.0 | ±3.5 | ⁻ |
| Group 2 | 0.005 | 209.6 | ±3.2 | ^{NS} | 221.0 | ±3.3 | ^{NS} |
| Group 3 | 0.015 | 212.8 | ±3.8 | ^{NS} | 219.6 | ±3.4 | ^{NS} |
| Group 4 | 0.05 | 211.8 | ±4.0 | ^{NS} | 219.8 | ±2.4 | ^{NS} |
| Group 5 | 0.15 | 215.4 | ±2.4 | ^{NS} | 222.8 | ±4.5 | ^{*} |
| Group 6 | 0.5 | 210.4 | ±2.6 | ^{NS} | 226.4 | ±4.6 | ^{**} |

| | | | | | | | |
|---|---|---|---|---|---|---|---|
| (Mean ± standard deviation, n = 4 or 5) **: There was a significant difference from group 1 (p < 0.01) *: There was a significant difference from group 1 (p < 0.05) NS: There was no significant difference from group 1 (p > 0.05) | | | | | | | |

[Table 2: Body length (from mouth to end of tail) after completion of 7-day repeated subcutaneous administration or 7-day continuous subcutaneous administration of CNP derivative (B) to 7-week-old male SD rat]

### CNP derivative (B)

**[Table 2]**

| Group | Dose | Body length (from mouth to end of tail) (mm) | | | | | |
|---|---|---|---|---|---|---|---|
| | | Repeated subcutaneous administration | | | Continuous subcutaneous administration | | |
| | (mg/kg/day) | Mean | ± standard deviation | | Mean | ± standard deviation | |
| Group 1 | 0 | 215.6 | ±2.6 | ⁻ | 211.6 | ±4.6 | ⁻ |
| Group 2 | 0.005 | 211.8 | ±2.9 | ^{NS} | 215.2 | ±3.5 | ^{NS} |
| Group 3 | 0.015 | 213.2 | ±4.4 | ^{NS} | 213.6 | ±4.4 | ^{NS} |
| Group 4 | 0.05 | 214.4 | ±4.7 | ^{NS} | 212.2 | ±5.2 | ^{NS} |
| Group 5 | 0.15 | 211.3 | ±4.5 | ^{NS} | 220.2 | ±4.3 | ^{*} |
| Group 6 | 0.5 | 215.2 | ±3.3 | ^{NS} | 222.8 | ±3.5 | ^{**} |

| | | | | | | | |
|---|---|---|---|---|---|---|---|
| **: There was a significant difference from group 1 (p < 0.01) *: There was a significant difference from group 1 (p < 0.05) NS: There was no significant difference from group 1 (p > 0.05) | | | | | | | |

### <Example 3> Effect duration of bone growth promoting effect of CNP derivative (A) in young rat

### [Method]

The CNP derivative (A) was dissolved in two types of vehicles differing in absorption rate from an administration site (vehicle 1: 0.03 mol/L acetate buffer solution (pH 4.0)/1% benzyl alcohol/10% sucrose, vehicle 2: 1% hyaluronic acid solution containing 0.1% L-methionine; solutions of the CNP derivative (A) dissolved in vehicle 1 and vehicle 2 are referred to as dosing solution 1 and dosing solution 2, respectively), and repeatedly subcutaneously administered at a dose of 0.5 mg/0.3 mL/kg or 0.25 mg/0.3 mL/kg once a day for 5 weeks to 5-week-old female Crlj:WI (Wistar) rats. Figure 3 shows their body lengths (from the mouth to the end of the tail) and lower limb tibial lengths on the final day of administration. Figure 4 shows change in estimated concentration in plasma during the repeated administration period.

### [Results]

The PK profile of the CNP derivative (A) differs largely by using two solvents differing in recipe as vehicles. In each case of using the solvents, a significant increase in body length (from the mouth to the end of the tail) and lower limb tibial length was observed as compared with rats at the same age without the administration of the CNP derivative (A) (normal control group). In the case of repeated subcutaneous administration at a dose of 0.5 mg/kg using vehicle 1, the estimated concentration in plasma was elevated to 200 ng/mL or higher immediately after administration, but decreased to 0.1 ng/mL or lower 9 hours after administration. On the other hand, in the case of repeated subcutaneous administration at a dose of 0.25 mg/kg using vehicle 2, the peak concentration in plasma was elevated merely to about 40 ng/mL, whereas the concentration was able to be kept at 0.1 ng/mL or higher even 12 hours after administration owing to slow disappearance. When increase in body length (from the mouth to the end of the tail) and lower limb tibial length was compared between these two conditions, it was evident that a stronger effect is obtained by using vehicle 2 which attained slow disappearance though the peak concentration in plasma was low. These results demonstrated that for the exertion of a bone growth effect induced by medication with the natriuretic peptide receptor-B (NPR-B) agonist, it is important to keep a concentration that exhibits agonistic activity for a long time, rather than to increase the peak concentration in plasma.

### <Example 4> Study on blood pressure lowering effect when CNP derivative (A) was continuously intravenously administered at constant rate for 24 hours to cynomolgus monkey

### [Method]

The CNP derivative (A) was continuously intravenously administered at doses of 0.015 to 0.1 mg/kg/day at a constant rate to telemetry transmitter-implanted cynomolgus monkeys (two males and two females), and studied for its influence on blood pressure (systolic, diastolic, and average blood pressures), heart rate, and an electrocardiographic parameter.

### [Results]

As for the systolic and average blood pressures, a blood pressure lowering effect exceeding 10 mmHg in terms of an average of differences from pre-administration values (reference for determination) was observed from 0.05 mg/kg. In the systolic blood pressure which showed the largest difference, -11.0 mmHg decrease and -20.5 mmHg decrease were observed for 0.05 mg/kg and 0.1 mg/kg, respectively (Figure 5 and Table 3). These pressure lowering effects are considered also to correlate well with the time-dependent change of the concentration in plasma of the CNP derivative (A). In this test, no symptom indicating reflex tachycardia was observed even when the concentration in plasma was increased to 9.8 ng/mL at its maximum. This is presumably because the blood pressure was very gradually decreased. These results suggested that: in waking cynomolgus monkeys, the risk of developing a decreased blood pressure can be drastically reduced by setting the concentration in plasma to 4 ng/mL or lower; and an adverse reaction such as reflex tachycardia is less likely to occur even if the concentration in plasma is elevated to about 10 ng/mL by continuous change.

[Table 3: Systolic blood pressure fluctuation range (maximum value during administration period) when CNP derivative (A) was continuously intravenously administered at constant rate for 24 hours to cynomolgus monkey, and concentration of CNP derivative (A) in plasma immediately before completion of administration

**[Table 3]**

| Drug | Dose (mg/kg/day) | Concentration in plasma (ng/mL) | Systolic blood pressure fluctuation range (maximum value %) |
|---|---|---|---|
| CNP derivative (A) | 0.015 | 0.4±0.1 | -6.8 |
| | 0.05 | 3.8±0.7 | -11.0 |
| | 0.1 | 9.8±2.6 | -20.5 |

### <Preparation Example 1> Preparation for subcutaneous infusion pump

The following preparation was produced as a preparation for the subcutaneous infusion pump for use in the present invention.
Pump: a syringe pump manufactured by TOP Corp. (indwelling needle type, syringe: made of polypropylene, tube: made of polyethylene)
Dosing solution: a 10 mM L-histidine buffer solution (pH 5.2) containing 5 mg/mL active ingredient (CNP derivative (A)), 10% sucrose and 0.3% metacresol

When the dosing solution was a freeze-dried preparation, a 10 mM L-histidine buffer solution (pH 5.2) containing 5 mg/mL active ingredient (CNP derivative (A)) and 10% sucrose was freeze-dried to produce a freeze-dried vial. Aside from this, a dissolving solution ampule of a 0.3% aqueous metacresol solution was provided. These were combined to obtain a kit preparation. Upon use, the dissolving solution was infused up to the amount of the solution before freeze drying to dissolve the freeze-dried product.

In the recipe described above, the CNP derivative (B) may be adopted instead of the CNP derivative (A) as the active ingredient.

## Claims

1. A therapeutic agent for failure-to-thrive, comprising CNP or a derivative thereof as an active ingredient, wherein the therapeutic agent, when administered to a human, is controlled such that the concentration in plasma of the active ingredient is kept in the range of 0.1 to 10 ng/mL for 8 hours or longer per day.

2. The therapeutic agent according to claim 1, wherein the CNP or the derivative thereof is a peptide consisting of the amino acid sequence of SEQ ID NO: 3 or 4.

3. The therapeutic agent according to claim 1, wherein the failure-to-thrive is any member selected from the group consisting of achondroplasia, hypochondroplasia, thanatophoric dysplasia, osteochondrodysplasia, Turner's syndrome, abnormal bone growth induced by steroid therapy, RAS/MAPK syndrome, growth hormone deficiency, growth hormone insensitivity syndrome, thyroid hormone deficiency, chronic renal failure, SHOX gene abnormality, neurofibromatosis type I, SGA short stature, mucopolysaccharidosis, and idiopathic short stature.

4. The therapeutic agent according to claim 1, wherein the concentration in plasma of the active ingredient is kept in the range of 0.1 to 4 ng/mL.

5. The therapeutic agent according to claim 1, wherein the duration of the concentration in plasma of the active ingredient per day is 16 hours or longer.

6. The therapeutic agent according to claim 1, wherein the therapeutic agent is in any form selected from the group consisting of an injection for drip infusion, a controlled-release microcapsule preparation, a controlled-absorption patch preparation for percutaneous administration, a gelled preparation, an isoelectric precipitation preparation, a complex formation preparation and a controlled-release subcutaneous injection preparation (SC infusion pump).

7. The therapeutic agent according to claim 6, wherein in the controlled-release subcutaneous injection preparation, the drug solution is a solution containing a CNP derivative consisting of the amino acid sequence of SEQ ID NO: 3 or 4, sucrose, and metacresol.


SEQUENCE LISTING

<110>  DAIICHISANKYO COMPANY, LIMITED

<120>  A drug for treating dwarfism

<130>  FP1806

<150>  JP2017-010736
<151>  2017-01-24

<150>  JP2017-089563
<151>  2017-04-28

<160>  5

<170>  PatentIn version 3.5

<210>  1
<211>  22
<212>  PRT
<213>  mammalian/homo sapience

<400>  1

Gly Leu Ser Lys Gly Cys Phe Gly Leu Lys Leu Asp Arg Ile Gly Ser
1               5                   10                  15

Met Ser Gly Leu Gly Cys
            20

<210>  2
<211>  53
<212>  PRT
<213>  mammalian

<400>  2

Asp Leu Arg Val Asp Thr Lys Ser Arg Ala Ala Trp Ala Arg Leu Leu
1               5                   10                  15

Gln Glu His Pro Asn Ala Arg Lys Tyr Lys Gly Ala Asn Lys Lys Gly
            20                  25                  30

Leu Ser Lys Gly Cys Phe Gly Leu Lys Leu Asp Arg Ile Gly Ser Met
        35                  40                  45

Ser Gly Leu Gly Cys
    50

<210>  3
<211>  39
<212>  PRT
<213>  Artificial

<220>
<223>  Chimeric CNP J (SEQ ID NO;157) of US2010-305031 and

WO2009/142307, and CNP derivative A of current invention.

<400>  3

Gly Leu Ser Lys Gly Cys Phe Gly Leu Lys Leu Asp Arg Ile Gly Ser
1               5                   10                  15

Met Ser Gly Leu Gly Cys Val Gln Gln Arg Lys Asp Ser Lys Lys Pro
            20                  25                  30

Pro Ala Lys Leu Gln Pro Arg
        35

<210>  4
<211>  39
<212>  PRT
<213>  Artificial Sequence

<220>
<223>  CNP derivative D, which disclosed in Am. J. Hum. Gent.(2012),
       91(6), 1108-1114

<400>  4

Pro Gly Gln Glu His Pro Asn Ala Arg Lys Tyr Lys Gly Ala Asn Lys
1               5                   10                  15

Lys Gly Leu Ser Lys Gly Cys Phe Gly Leu Lys Leu Asp Arg Ile Gly
            20                  25                  30

Ser Met Ser Gly Leu Gly Cys
        35

<210>  5
<211>  17
<212>  PRT
<213>  Artificial

<220>
<223>  A ring structure portion of CNP

<220>
<221>  MISC_FEATURE
<222>  (11)..(12)
<223>  the amino acid could be Ser or Ala on position 11 and Met, Phe or
       Glu

 on position 12

<400>  5

Cys Phe Gly Leu Lys Leu Asp Arg Ile Gly Xaa Xaa Ser Gly Leu Gly
1               5                   10                  15

Cys